(19) Europäisches Patentamt
European Patent Office
Office européen des brevets

(11) **EP 2 689 226 B1**

(12) **EUROPEAN PATENT SPECIFICATION**

(45) Date of publication and mention
of the grant of the patent:
**26.06.2019 Bulletin 2019/26**

(51) Int Cl.:
*G01L 11/02* (2006.01)        *G01L 9/00* (2006.01)
*A61M 5/142* (2006.01)      *A61M 5/168* (2006.01)

(21) Application number: **12709891.1**

(22) Date of filing: **22.03.2012**

(86) International application number:
**PCT/EP2012/055072**

(87) International publication number:
**WO 2012/126980 (27.09.2012 Gazette 2012/39)**

(54) **SENSOR DEVICE FOR USE IN A MEDICAL FLUID DELIVERY SYSTEM**

SENSORVORRICHTUNG ZUR VERWENDUNG IN EINEM MEDIZINISCHEN FLÜSSIGKEITSABGABESYSTEM

DISPOSITIF DE DÉTECTION UTILISABLE DANS UN SYSTÈME MÉDICAL D'ADMINISTRATION DE FLUIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.03.2011 EP 11159202**

(43) Date of publication of application:
**29.01.2014 Bulletin 2014/05**

(73) Proprietors:
• **F. Hoffmann-La Roche AG**
**4070 Basel (CH)**
Designated Contracting States:
**AL AT BE BG CH CY CZ DK EE ES FI FR GB GR HR HU IE IS IT LI LT LU LV MC MK MT NL NO PL PT RO RS SE SI SK SM TR**
• **Roche Diabetes Care GmbH**
**68305 Mannheim (DE)**
Designated Contracting States:
**DE**

(72) Inventors:
• **GEIPEL, Andreas**
**68542 Heddesheim (DE)**
• **GROSSENBACHER, Pascal**
**CH-3006 Bern (CH)**
• **MICHEL, Philipp**
**CH-3052 Zollikofen (CH)**
• **HAUETER, Ulrich**
**CH-3506 Grosshöchstetten (CH)**

(74) Representative: **Rentsch Partner AG**
**Bellerivestrasse 203**
**Postfach**
**8034 Zürich (CH)**

(56) References cited:
**FR-A1- 2 540 987    US-A1- 2009 118 667**

• **KOHL M J ET AL: "A microfluidic experimental platform with internal pressure measurements", SENSORS AND ACTUATORS A, ELSEVIER SEQUOIA S.A., LAUSANNE, CH, vol. 118, no. 2, 28 February 2005 (2005-02-28), pages 212-221, XP025324936, ISSN: 0924-4247, DOI: DOI:10.1016/J.SNA.2004.07.014 [retrieved on 2005-02-28] cited in the application**

## Description

## Field of the Invention

The invention relates to a sensor device for use in a medical fluid delivery system, in particular for an infusion pump device, like an insulin infusion pump device, to monitor a fluidic pressure in a fluidic system of such system and device. Furthermore the invention relates to a method for monitoring the pressure and/or detecting occlusions in a fluidic system.

## Background of the invention

Medical fluid delivery systems for the administration of e. g. liquid medicament fluids, such as infusion pump devices, are often used with patients who have a continuous, and in the course of the day varying, need of a medicine that can be administered e. g. by subcutaneous infusion. Specific applications are for example certain pain therapies and the treatment of diabetes. In such cases, for example computer controlled automated infusion pump devices are used, which can be carried by the patient on the body, and which contain a certain amount of liquid medicament in a medicine reservoir. The liquid medicament is supplied to the patient's body from the medicine reservoir through a fluidic system to an infusion cannula or an injection needle. Mostly such medical fluid delivery systems comprise a reusable unit including an actuation mechanism, a dosing mechanism, electronics for controlling the mechanisms, etc. and a disposable unit e. g. including a fluid reservoir, which is discarded after emptying the reservoir. Such an Infusion pump device is for example explained in EP 1970677A1.

The liquid medicament is e. g. obtained by a downstream pump from a flexible container. Flexible containers have the advantage of a smaller volume surplus of the container in relation to its content, which reduces the manufacturing costs and enables design of infusion pump devices of smaller overall dimensions. Thus the device is not evident through clothing and can be carried as comfortably as possible.

In the context of liquid medicament administration via an infusion pump device, sensor devices are used for controlling the dosing, monitoring the correct operation of the system, and for fast detection of faults and hazards, such as occluded infusion lines or cannulae, empty containers, or malfunctioning pump systems. A pressure sensor device may be arranged in a fluid path downstream of a pump device and upstream of an infusion cannula.

Such pressure sensor devices typically comprise a micro-fluidic chamber filled with liquid and fluidly connected to the fluidic system. The chamber is covered by a flexible, resilient membrane, such that a pressure difference between the fluidic pressure inside the sensor chamber and the outside (such as atmospheric) pressure will temporarily deform the membrane. The resulting deflection of the membrane can then be measured by suitable means in order to determine the internal pressure of the fluidic system.

A suitable approach to measure the deformation of the membrane is optical detection of a light beam reflected on the membrane. Such pressure sensor devices are, for example, disclosed in "A microfluidic experimental platform with internal pressure measurements", M. J. Kohl et al., Sensors and Actuators A 118 (2005), pp. 212-221 . The pressure sensor device includes a micro-fluidic chamber that is connected to a fluidic system comprising a rigid bottom substrate and a flexible, resilient top cover, for example, a membrane. An optical detection system is arranged to measure a deformation of the cover membrane by determining the interaction of a light beam with the cover membrane. For that purpose a light emitting device, e.g. a laser diode, directs a light beam at a certain angle onto the surface of the cover membrane, where it is reflected. The pressure difference between the inner volume of the micro-fluidic chamber and the outer environment acts on the cover membrane, and deforms it to a certain extent, depending on the pressure difference. As a result the angle of the reflected light beam changes and the beam is transversely shifted. By monitoring the position of the reflected light beam, the deformation of the cover membrane can be measured, and based on the obtained results a pressure difference value can be determined.

In WO 2007/093064 A1 an insulin pump is shown, which comprises a reusable unit with drive, dosing and controlling systems and a disposable unit with a flow path and a catheter tube. The flow path can be connected to the driving and dosing mechanism, such that a liquid system runs from a liquid reservoir to the catheter tube. A resilient membrane is arranged in the flow path and covers a fluidic chamber. The membrane is impinged directly by the liquid in the flow path, such that it is deflected towards the reusable unit in case of a pressure increase e. g. due to an occlusion of the liquid system. The reusable unit comprises a light emitter in form of a laser diode and a photosensitive x-y sensor, which is connected to the controlling system. The light beam of the laser diode can be directed on a rigid reflector arranged on the membrane. Alternatively the light beam can be directed directly on the membrane surface. In this case a small area on the surface of the membrane is metal-coated, to provide good reflecting properties of the membrane. The incident light beam is focused on an area of the membrane, which is distanced to the center of the membrane. That means in a deflected state of the membrane the reflecting area is inclined and rounded with respect to the non-deflected state. Therefore a reflected light beam changes direction and shape, which can be detected by the x-y sensor, and which is an indication for a pressure change within the liquid system.

An other infusion pump system is disclosed in WO 2008/144693 A1, which comprises an occlusion sensor that can be used to detect when an occlusion exists in the fluid path between a medicine reservoir and the infusion site. The occlusion sensor includes a flexible membrane, which can be deflected by rising pressure in the fluid path, such that it touches a wall part of an air cavity. The incident measurement light beam is guided through a light transmissive member to the air cavity. In a non-deflected state the incident beam is totally reflected at the air cavity. In a deflected state, when the membrane touches the air cavity, reduced reflection occurs. The reduction of the intensity of the reflected light beam is a measurement for the pressure in the fluid path.

The FR 2540987 A1 discloses an electro-optical pressure sensor that is realized as compact integral unit and is based on focusing a light beam on an inflexion point area of a pressure-deformable membrane and receiving a reflected light beam by a sensor which is arranged such that the reflected light beam is, in the absence of pressure deformation of the membrane, focused on the sensor center.

Since some of the parts of the pressure sensor device are arranged in the disposable unit and other parts are arranged in the reusable unit the orientation of disposable and reusable part towards each other is a critical parameter of reliable measuring results. In the known systems the tolerances between these units must be as small as possible. For being able to monitor the reflected light beam in a pressure sensor according to the prior art, a detector in the optical detection system often is designed to be movable, or a multiplicity of detectors at different positions and at different angles are included in the device. Both of these aspects make such sensor devices expensive and difficult to make. A wide range of directions of reflected light beams due to the large variety or diffuse reflection angles on a membrane also requires a complex sensor array in the pressure sensor device.

Therefore there is a general need for improvements in this area.

## Objects of the Invention

It is an object of the present invention to provide an improved sensor device for use in a medical fluid delivery system, particularly in an infusion pump device for liquid medicaments, like insulin, which overcomes the drawbacks of prior art devices.

It is a further object of the present invention to provide a medical fluid delivery comprising a sensor device, that fulfills above mentioned objects. It is still a further object of the present invention to provide a method for monitoring pressure variations in a medical fluid delivery system, which is easy to install and indicates pressure changes or fluid path occlusion in a simple manner.

These and other objects are substantially achieved through the features of the independent claims. In addition, further advantageous embodiments follow from the dependent claims and the description. Further advantageous embodiments follow from the dependent claims and the description.

## Summary of the invention

A sensor device according to the present disclosure for use in a medical fluid delivery system comprises a fluidic chamber with a deformable cover closing at least one area of the chamber and an optical detection system comprising at least one light emitter for emitting one or more incident light beams and a sensor unit for monitoring one or more reflected light beams. In a pressurized state of the fluidic chamber the deformable cover is de-formed such that it forms an inflexion point area within the deformed cover. The one or more incident light beams emitted by the light emitter are directed on the cover such that the one or more incident light beams are reflected essentially in the inflexion point area on the cover located closest to the light emitter.

The fluidic chamber can be realized as part of a fluid path of a medical fluid delivery system. It can be formed e. g. as a micro-fluidic chamber, which is in fluidic connection with such a fluid path. A fluidic chamber may e. g. be designed as described in EP 2399626 A1 where appropriate for a sensor device according to the present disclosure. The cover can be placed over an opening in the fluidic chamber, such that the cover is in contact with the interior atmosphere of the chamber. Advantageously, the cover covers an at least partially rounded opening of the fluidic chamber. The cover may be in direct contact with a fluid delivered through the fluidic chamber. But in general it is possible to locate a transmission volume, like a gas volume, between the fluid and the cover, which transmits pressure changes in the fluid to the cover.

In one embodiment of the disclosure the fluidic chamber is part of a disposable unit of a medical fluid delivery system and the optical detection system is part of a reusable unit of the medical fluid delivery system. The light emitter is for example provided by one or more light emitting diodes (LED) or laser diodes. Advantageously one such diode emitting one light beam of one wavelength is enough to realize the sensor device. The sensor unit can for example be provided as photodiodes.

The cover e. g. is a flexible, resilient membrane cover, which is deformable or deflectable respectively. In a normal state of operation of the sensor device or the medical fluid delivery device, that means a regular pressure atmosphere within the fluidic chamber, the cover in general is essentially not deformed. In case of a medical fluid delivery system for insulin such a regular pressure is e. g. 200 to 2000 mbar relative pressure. This state will be indicated as a non-pressurized state of the fluidic chamber, although a specific normal pressure always exists in the system. The non-pressurized state can be determined by a reference measurement with the optical detection system. Thus the non-pressurized state may serve as a reference state and pressure changes are detected in respect to this reference state. In case the pressure within the fluidic chamber increases or decreases, the cover will be deformed due to the force of the pressure change. This state will be indicated as a pressurized state of the fluidic chamber comprising a deformed cover.

The inflexion point area is characterized as the area wherein the radius of curvature of the cover changes along a line running within the cover from an edge of the cover connected to the fluidic chamber to the center of the cover. It can also be characterized as the area within the absolute value of the curvature changes sign. In this area the curvature of the cover is negligible in approximation and acts as an even or nearly even reflection area. In fact when the cover is deformed and buckling to one or the other side an inflexion line around the center of the cover is formed.

The incident one or more light beams are directed on the cover at an angle $\alpha$. In the normal state of the cover, i.e. the non-pressurized state, the incident light beam meets the cover in an area, which corresponds to a potential inflexion point, or in close vicinity of this area. While the fluidic chamber is pressurized to a pressurized state that area is tilted and mostly also offset translative compared to the normal state due to the deformation of the cover. Since the area comprises the inflexion point, it is approximately even and may act as plane reflection area for the incident light beam in the pressurized state. At the same time it is the area with the most angular change compared to the normal state of the cover. That means the one or more light beams are reflected in the area of the cover comprising the largest change of orientation towards the incident light beams, when passing from the non-pressurized state to the pressurized state. But around the inflexion point there is a minimum sphericity negatively affecting the quality of the reflected light beam.

The sensor device according to the disclosure allows a maximum change in reflection of the one or more incident light beams in the pressurized state compared to the non-pressurized state of the cover and therefore ensures accurate observation of pressure abnormalities within the fluidic chamber. The detection at the maximum deflection of the reflected light beams enables a simple and cost-efficient construction of the sensor unit and provides large flexibility in the arrangement of the elements of the sensor device relative to each other.

Good results are achieved by using an incident light beam, which is essentially a parallel light beam. Therefore a laser diode or a light emitting diode comprising an optical element for parallelizing emitted light beams can be used advantageously. In case of a parallel incident light beam and the fact, that the deformed cover is generally planar in the inflexion point area the reflected light beam also is basically parallel and can be focused precisely towards the sensor unit.

Depending on the light emitter used the incident light beam may practically be realized by a bundle of light beams, which covers not only a point on the cover but rather spreads over an area on the cover surface. The diameter of the over all incident light beam should be selected such that an edge of the beam does not extend beyond the center point of the cover on the fluidic chamber. The diameter of the incident light beam may be 1/3 or less of the cover diameter. It is advantageous if it is less than 1/4 and even less than 1/5 of the cover diameter.

In a non-pressurized state of the fluidic chamber the incident one or more light beams can be directed on the cover at an angle $\alpha$. Alternatively the incident light beams may be focused perpendicular on a non-pressurized cover. An angled direction provides large flexibility in the construction of the single elements of the optical detection system.

Due to the optimized deflection of the incident light beams by the inflexion point area of the fluid chamber cover it is not necessary to provide a sensor unit with a large array of photodiodes. In an advantageous embodiment of the disclosure the sensor unit is provided by a dual-element sensor, which comprises two photo-elements. For example two photodiodes can be arranged directly next to each other in one line. The incident light beam can be focused on a first photodiode, when the fluidic chamber is in a non-pressurized state, and is focused on a second photodiode, when the fluidic chamber is in a pressurized state. That means while a pressure change in the fluidic chamber occurs, the reflected light beam wanders from the first photodiode to the second photodiode. From a particular pressure value on the reflected light beam is fully focused on the second photodiode. The particular pressure value is adapted to correspond to a specific application of the sensor device. For the use of the sensor device as a pressure sensor e. g. in an infusion pump device, the reflected light beam shall change from first to second photodiode within a range of pressure change of 200 mbar to 2000 mbar. Also the material of the cover or the arrangement of the two photodiodes may be adapted to relevant pressure changes and the particular pressure value respectively.

Alternatively the two photodiodes may be arranged separated from each other. In this case at least one optical element may be provided in an optical path from the inflexion point area towards one or both of a first and/or a second photodiode, to direct the reflected light beam to the respective photodiode. The optical element or elements are located such that in a non-pressurized state the reflected light beam hits the first photodiode directly or a first optical element directs the beam to the first photodiode. In a pressurized state the reflected light beam is deflected by the inflexion point area of the cover such that it moves away from the first photodiode or the first optical element and instead hits a second optical element, which guides the beam to the second photodiode, or meats the second photodiode directly. A mirror element can for example be used to guide the reflected light beam.

In one embodiment the light emitter of the sensor device according to the disclosure is located on a side of the cover which is curved in a convex manner in an increased pressure state of the fluidic chamber. That means the light emitter directs the one or more incident light beams directly on the cover instead of first passing the interior of the fluidic chamber and the fluid delivered through the chamber. Advantageously one side of the cover is in fluid contact and the light emitter is located on the other side. In this arrangement the point of incidence of the incident light beam, which defines the inflexion point area on the cover, is subject to a superposition of two effects when the cover changes from the normal state to the pressurized state and shaping the inflexion point or line, as mentioned above. First the point of incidence is elevated translational by a distance w and second the point of incidence is tilted by an angle ε. That means in the deformed state of the cover the inflexion point area changes its angular and its translational location in respect to the non-deformed state. A distance s travelled on the sensor unit by the reflected light beams, e. g. from the first photodiode to the second photodiode, can therefore be determined by the superposition in case of small elevation and deflection:

$$s = 2 \cdot w \cdot \cos(\alpha) + d \cdot \tan(2\,\varepsilon),$$

wherein $\alpha$ is defined by the angle of incidence between the incident light beam and the cover and d is the distance from the point of incidence on the cover to sensor unit. The angle $\alpha$ may lie between 30° to 60° and even between 40° to 50°. In this range the distance s acts basically linear to a pressure within the fluidic chamber.

Of course the degree of tilting and elevation depends on the material used for the cover or membrane cover respectively. The cover can be realized as a polymer foil. The polymer foil may be an integral part of the disposable unit of a delivery device or the like. Material used is for example PMMA, PC, PET, PA, PSU, or COC/COP. The thickness of the cover material is for example between 50 m and 200 m, advantageously between 80 m and 150 m. The cover must reflect a sufficient portion of the incident light beam from the light emitter. Therefore the cover material can be metalized, for example by a layer of aluminium, chrome, silver, gold or the like. Alternatively a metal foil comprising sufficient elasticity may be used. According to yet another aspect the disclosure refers to a medical fluid delivery device, which comprises a sensor device according to the disclosure as mentioned above. As described the delivery device may comprise a reusable unit in combination with a disposable unit. The disposable unit comprises for example a fluid reservoir and usually is discarded after use. The reusable unit is designed such that a disposable unit can be connected to it. Also the sensor unit is may be located in the reusable unit. Advantageously the optical detection system of the sensor device is part of the reusable unit of the delivery device and the fluidic chamber is part of the disposable unit of the delivery device. The disposable unit may comprise an optical element for directing the incident light beam in a predetermined angle

$\alpha$ on the inflexion point area of the cover. That means the light emitter can be arranged in any suitable manner relative to the fluidic chamber and the cover respectively, e. g. such that the emitted light is orientated parallel or perpendicular to the cover. The parallel or perpendicular light beam is refracted by the optical element such that light beam is directed to the cover in the desired angle $\alpha$. Furthermore the disposable unit of the delivery device may comprise an optical element for directing the reflected light from the inflexion point area to the sensor unit as discussed before.

In one embodiment of the medical fluid delivery device according to the disclosure the optical elements for the light path of the incident and/or reflected light beams may be part of the disposable unit of the delivery device. For example the optical elements may be part of a cap or covering of the fluidic chamber, which is attached to a surrounding edge of the cover of the fluidic chamber. The optical elements are transparent for the light of the light emitter. The optical elements are made of amorphous polymer material for example. The fluidic chamber and the optical elements may be made of the same material. With the use of such a disposable unit, the incident light beam of the light emitter can be orientated perpendicular to the cover on the fluidic chamber. The perpendicular light beam is deflected by the first optical element or the covering of the disposable unit such that the incident light beam is directed in angle $\alpha$ on the inflexion point area of the cover. After reflection of the light beam a second optical element may focus the reflected beam onto the sensor unit or one of the photodiodes of the sensor unit respectively.

The use of optical elements facilitates a correct positioning of the disposable and reusable units relative to each other. The optical elements can compensate a mismatch of the units within a tolerance of approximately $\pm 0.5$ mm. Therefore a medical fluid delivery system comprising a sensor device according to the present disclosure enables accurate observation of the functioning of the delivery system.

According to a method according to the present disclosure, for monitoring a pressure change in a fluidic chamber of a medical fluid delivery system according to the disclosure using a sensor device as described before one or more incident light beams are directed on the inflexion point area of the deformable cover. One or more light beams reflected from the deformable cover in a non-pressurized state and a pressurized state are detected. The detection data of the non-pressurized state and the pressurized state are compared to extract the pressure change value. Therefore the optical detection system can be connected to an electronic controlling system of the medical fluid delivery system. A threshold pressure level can be defined and deposited in the controlling system, at which the delivery system is shut down or an alarm is given to indicate a critical pressure value or malfunction of the system. In case of an occlusion of a fluidic path of the medical fluid delivery system for example the threshold value may be between 200 mbar and 2000 mbar relative to the atmosphere, mostly it is between 500 mbar and 1200 mbar and it even can be around 1000 mbar.

In a non-pressurized state of the fluidic chamber a reflected light beam reflected from the non-deformed cover of the fluidic chamber can be directed on a first photo-element of the dual-element sensor and in a pressurized state of the fluidic chamber a reflected light beam reflected from the deformed cover can be directed on a second photo-element of the dual-element sensor. The threshold pressure level may e. g. be reached as soon as the reflected beam fully left the first photo-element and fully hits the second photo-element. Alternatively the threshold pressure level may be achieved as soon as a particular value of light intensity is detected on the second photo-element. In a still further alternative, the difference of the light intensity detected by the two photo-elements is evaluated.

The sensor device according offers reliable and simple detection of pressure changes and/or occlusion in a fluid path of the system. It allows a space saving structure of a delivery system and reduces complexity of detection algorithms. Also the sensor device can easily and safely be used in combination with reusable and disposable units of the system and can be produced cost-efficiently.

**Brief description of the drawings**

Advantageous exemplary embodiments of the invention will be described in the accompanying drawings, which may explain the principles of the invention but shall not limit the scope of the invention. The drawings illustrate:

Figure 1a:  schematic cross-sectional view of a sensor device according to the present disclosure in a non-pressurized state,

Figure 1b:  schematic cross-sectional view of the sensor device according to figure 1a in a pressurized state,

Figure 2a:  schematic illustration of a translational offset of the cover in a pressurized state,

Figure 2b:  schematic illustration of an inclination of the cover in a pressurized state,

Figure 2c:  schematic illustration of a superposition of the translational offset and the an inclination of the cover in a pressurized state,

Figure 3a:  diagram of sensor signals of a sensor device using sensor unit comprising a dual-element sensor,

Figure 3b:  diagram of an algorithm to determine a threshold pressure value in accordance to a reference pressure value,

Figure 4:  schematic illustration of a first embodiment of a sensor device according to the disclosure using optical elements to direct incident and reflected light beams,

Figure 5:  schematic illustration of a second embodiment of a sensor device according to the disclosure using optical elements,

Figure 6:  schematic illustration of a third embodiment of a sensor device according to the disclosure using optical elements,

Figure 7:  schematic illustration of a fourth embodiment of a sensor device according to the disclosure using optical elements for splitting reflected light beams,

Figure 8:  schematic illustration of a fifth embodiment of a sensor device according to the disclosure using optical elements for splitting reflected light beams,

Figure 9:  schematic cross-sectional view of a first embodiment of a covering of a disposable unit of a medical fluid delivery system according to the disclosure comprising optical elements to direct incident and reflected light beams, and

Figure 10:  schematic cross-sectional view of a second embodiment of a covering of a disposable unit of a medical fluid delivery system comprising optical elements.

**Description of embodiments of the invention**

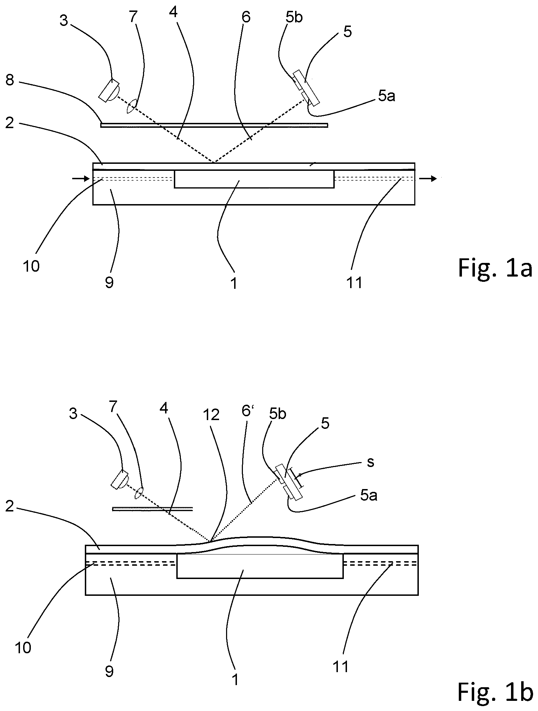

In figures 1 to 10 the basic elements and the function of a sensor device and a method for monitoring pressure changes in a medical fluid delivery system according to the disclosure are explained with reference to schematic illustrations showing features of the disclosure. The general structure of medical fluid delivery systems or infusion pump devices is know from prior art, e. g. from EP 1970677 A1 or WO 2007/093064 A1. Thus in respect of the general function of a medical fluid delivery system it is referred to this disclosure. Figure 1a shows a schematic cross-sectional view of a sensor device for use in a medical fluid delivery system according to the present disclosure. The sensor device comprises a fluidic chamber 1 with a deformable cover 2 closing at least one area of the chamber. An optical detection system of the sensor device comprises a light emitter 3, an LED in this case, for emitting incident light beams or a bundle of light beams 4 and a sensor unit 5 for monitoring one or more reflected light beams 6. An optional optical element 7 in form of a collective lens, an aperture, or the like is arranged in the light path of the incident light beam 4 to parallelize the emitted light of the LED 3. The sensor unit 5 in form of a dual-element sensor comprises a first photodiode 5a and a second photodiode 5b, which are arranged directly next to each other in this embodiment. The optical detection system is arranged in a reusable unit of a medical fluid delivery system, wherein the incident light beam and the reflected light beam pass through an optical window 8 of the reusable unit to the exterior of the reusable unit.

The fluidic chamber 1 is part of a fluidic module 9 comprising a cavity, which is covered by the deformable cover 2 on one side. The fluidic chamber 1 has the form of a microfluidic chamber. A fluid inlet 10 connects the fluidic chamber 1 to a fluid reservoir (not shown) and a fluid outlet 11 leads from the fluidic chamber 1 to a patient. The fluid inlet 10, the fluidic chamber 1 and the fluid outlet 11 are part of a fluidic path leading from the fluid reservoir to the patient. The cover may e. g. be glued or welded to the surface of the fluidic module 9. The cover 2 covers an at least partially rounded opening of the fluidic chamber 1. Mostly the opening or cavity is fully circular, which facilitates detecting the cover in a pressurized state and establishing a gentle curvature of the cover. The fluidic module 9 is part of a disposable unit of the medical fluid delivery system, which can be attached to the reusable unit.

In figure 1a the fluidic chamber 1 is in a non-pressurized state, which means the pressure value in the chamber corresponds to normal operation of the medical fluid delivery system as explained before. The deformable cover 2 is not deflected but stretching planar over the fluidic chamber 1. The incident light beam 4 is reflected on the planar surface of the deformable cover 2, such that the reflected light beam 6 is directed on the first photodiode 5a of the sensor unit 5. The photodiode 5a sends a signal to an electronic control system (not shown) of the medical fluid delivery system, which indicates a normal pressure condition within the fluidic path.

In figure 1b the fluidic chamber 1 is shown in a pressurized state. As shown the pressure within the fluidic chamber 1 increased such, that the deformable cover 2 is deformed and forms an inflexion point area 12 within the cover surface covering the fluidic chamber. The deformed cover 2 has a convex shape with respect to the optical detection system and the reusable unit respectively, when the pressure within the fluidic path increases. The incident light beam 4 emitted by the light emitter 3 is directed on the cover 2 such that the incident light beam 4 is reflected essentially in the inflexion point area 12, mostly exactly in the inflexion point. The reflected light beam 6' is deflected due to the change in orientation of the deformable cover 2 in respect to the non-deformed cover by a distance s. The reflected light beam 6' now is directed on the second photodiode 5b of the sensor unit 5. The photodiode 5b sends a signal to the electronic control system, which indicates a pressure change in the fluidic chamber 1 and therefore in the fluidic path.

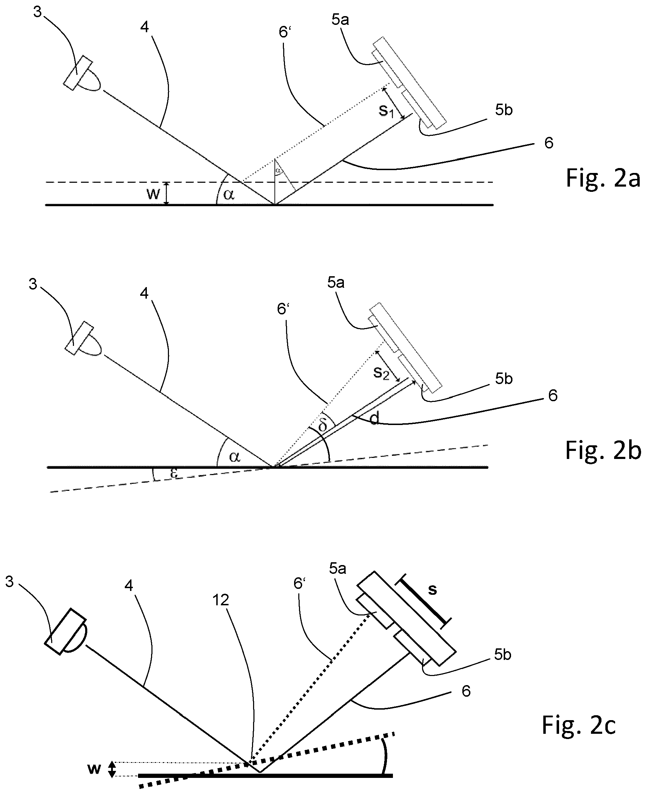

In figures 2a to 2c the change in reflection of the incident light beam 4 due to the deformation of the cover 2 is explained in more detail. In comparison to the normal non-pressurized state in the pressurized state the deformable cover is lifted translational by a height w, as shown in figure 2a. That means the reflected light beam is shifted and offset by a distance $s_1$, which is defined by $s_1 = 2 \cdot w \cdot \cos(\alpha)$, wherein $\alpha$ is the incident angle between the incident light beam and the deformable cover. The reflected light beam 6' of the pressurized state is shifted from the first photodiode 5a in direction of the second photodiode 5b by the distance s1. Also the deformable cover 2 is tilted by an angel $\varepsilon$ relative to the non-pressurized state, which changes the incident angle of the incident light beam, as shown in figure 2b. In the inflexion point area the curvature of the deformed cover changes. The surface of the deformed cover is approximately even and the angle $\varepsilon$ is approximately constant in this area. The direction of the reflected light beam is changed by an angle $\delta$, which is defined by $\delta = 2 \cdot \varepsilon$. That leads to a shift $s_2$ on the sensor unit 5 from the first photodiode 5a in direction of the second photodiode 5b, wherein $s_2 = d \cdot \tan(2\varepsilon)$ and d corresponds to the distance between the reflection point on the deformed cover and the sensor unit 5. As shown in figure 2c, the total shift s of the reflected light beam 6 of the non-pressurized state to a reflected light beam 6' of the pressurized state by a deformation of the cover 2 is given by $s = s_1 + s_2$.

This corresponds to a linear mode of calculation, wherein the total shift s is linear in relation to the pressure in the fluidic chamber 1 approximately. Of course more sophisticated calculation modes may be used. Also the mode of calculation is based on a point-shaped light beam. In practice, in particular when using an LED as light emitter 3, the light beam is rather a bundle of parallel light beams comprising a diameter $D_{lb}$. For using the shown calculation the beam diameter $D_{lb}$ should be selected such, that light at the edge of the bundle does not extend over the center point of the deformable cover 2. The maximum bundle diameter $D_{max}$ should fulfill the following requirement: $D_{lb}$ 0.58 sin $(\alpha)$ $D_{cov}$, wherein $D_{cov}$ is the diameter of the deformable cover 2. The diameter $D_{cov}$ is measured from a center point of the deformable cover in direction to the inflexion point area chosen for reflecting the incident light beam.

The dual-element sensor 5 generates two separated singular raw signals $S_1$ and $S_2$, one signal $S_1$ of the photodiode 5a and a second signal $S_2$ of the photodiode 5b. In case of a point-shaped light beam, e. g. as emitted by a laser diode, the dual element sensor generates a binary sensor signal S, i. e. one of the raw signals is on and the other one of the raw signal is off. This is because only one of the photodiodes 5a and 5b is energized by the point-shaped light beam at a time. When using a LED as light emitter 3 comprising a bundle of parallel light beams with a diameter $D_{lb}$ a gradual shift from one photodiode 5a to the other photodiode 5b occurs as explained above.

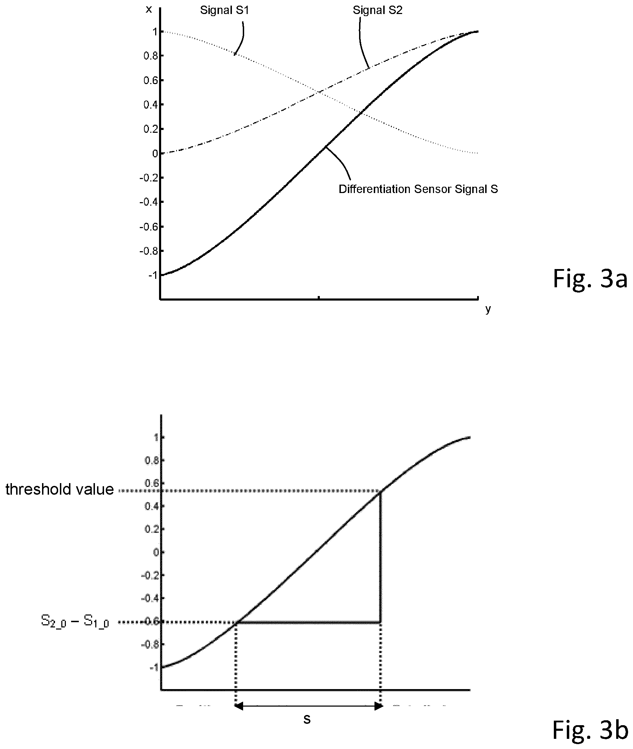

Figure 3a illustrates the characteristics of the raw signals $S_1$ and $S_2$ and a differentiation signal S when a center of the beam bundle is shifted from one center point on photodiode 5a to the adjacent center point on photodiode 5b during deformation of the cover 2, wherein the y-axis indicates the value of the sensor signals and the x-axis indicates the position of the center of the light bundle on the sensor 5. While the beam bundle is centered on the first photodiode 5a raw signal $S_1$ has the value 1 and raw signal $S_2$ has the value 0. During the deformation of the cover 2, the beam bundle wanders to the center of the second photodiode 5b, where raw signal $S_1$ has the value 0 and raw signal $S_2$ has the value 1. By differentiation $S_2 - S_1$ of the two raw signals the sensor signal S is extracted, which is sufficiently linear in the area of transition between the two photodiodes. Such calculation model allows a quantitative determination of the pressure values in the fluidic chamber.

In practice the exact development of the sensor signal S and the behavior of the reflected light beam depends on the relative positioning between the disposable unit and the reusable unit. The positioning usually lies within a tolerance of ± 0.2mm and may be different for each disposable unit. Therefore after placing a new disposable unit on the reusable unit the sensor signal S is taken as a reference value, wherein the fluidic pressure on the deformable cover is 0 bar relative to the atmosphere. The reference value allows the integration of the absolute position of the cover before starting the operation of the medical fluid delivery system in the calculation model. Afterwards the usual operation of the system starts for example by priming the fluidic path of the system with fluid.

In case the sensor device according to the present disclosure is used as an occlusion sensor for detecting occlusion of the fluidic path, e. g. for a medical fluid delivery system, a characteristic occlusion threshold value is defined, which indicates an occlusion. In a simple algorithm the threshold value may be defined as a fixed value. Alternatively the threshold value of the differentiation sensor signal is provided as a function of the reference value. Thus an influence of disturbing factors, like temperature effects or tolerance variations, can be minimized, which means the threshold value corresponding to occlusion is varied as little as possible. One possibility is to calculate the threshold value of the differentiation sensor signal for each disposable unit at the beginning of the use of the disposable unit by involving the reference value and finally save the threshold value as fixed value. Again the characteristics according to figure 3a are used to describe the sensor signal S. An expected shift s (see above) of the reflected light beam, which corresponds to an occlusion, is known. Therefore based on the reference value, the expected shift s and the characteristics of the sensor signal S the threshold value of the differentiation sensor signal for the occlusion can be determined as shown in figure 3b. The corresponding occlusion requirement is:

$$S_2 - S_1 \geq (S_{2\_0} - S_{1\_0}) + m \cdot s$$

wherein $S_1$ is the raw signal of the first photodiode, $S_2$ is the raw signal of the second photodiode, $S_{1\_0}$ is the reference value of the first photodiode at 0 bar, $S_{2\_0}$ is the reference value of the second photodiode at 0 bar, m is the inclination of the characteristics of the sensor signal S in a linear range, and s is the expected shift of the reflected light beam on the sensor unit.

Another possibility to determine the threshold value of the differentiation sensor signal is to save the reference value of the specific disposable unit and calculate the threshold value at the time of detection.

Non-linear effects in determining an occlusion threshold value can be minimized for example by involving results of experimental investigations. Experiments have shown, that a required correction of a threshold value is bigger, the higher the difference between a reference value and a nominal value corresponding to an ideal situation is. One possible algorithm for defining an occlusion pressure value based on experimental investigations involves only the reference value $S_{1\_0}$ of the first photodiode and is described by a hyperbolic relation as follows:

$$S_2 - S_1 \geq (a / S_{1\_0}) + c$$

wherein a corresponds to an empirical weighting and c corresponds to an offset value. The weighting factor a minimizes unwanted non-linear factors in the relevant range of the sensor signal characteristics. The offset value c controls the desired threshold value. The weighting factor a and the offset value c have to be adapted to a specific design of the sensor device taking into account different basic conditions, e. g. thickness of the cover, diameter of the cover, reflectivity of the cover, etc.. Mostly the threshold value for an occlusion is within the range of 300 mbar and 1500 mbar. Furthermore a temperature factor can be taken into consideration. A temperature sensor can be used to determine temperature changes, which can be located close to the interface between the disposable and reusable unit. Alternatively a forward voltage of the LED may be used as temperature sensitive value.

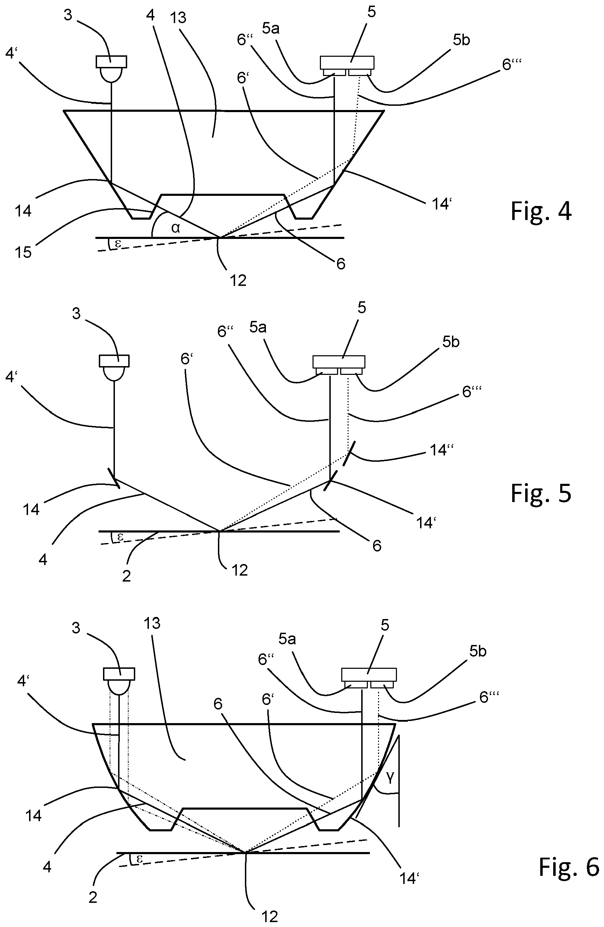

In figures 4 to 8 different embodiments of a sensor device according to the present disclosure are shown, wherein a light emitter 3 does not focus an incident light beam 4 directly on the deformable cover 2 but one or more optical elements are provided, for example as part of a disposable unit of a medical fluid delivery system, which deflect the light beam 4' of the light emitter and directs it as the incident light beam 4 in a predetermined angle on the deformable cover. The incident light beam may reach the deformable cover in an angle between 30° and 60° and mostly in an angle between 40° and 50°. Furthermore an optical element is provided in an optical path from the inflexion point area 12 towards one or both of first and/or second photodiodes. These optical elements deflect the reflected light beam 6 of a non-pressurized state and the reflected light beam 6' of a pressurized state such that they are directed to the sensor unit. The optical elements may be designed as separate elements or realized in an optical unit including several optical elements. The material of the optical elements or unit is mostly transparent, for example an amorphous polymer may be used. Advantageously the optical elements or unit are made of the same material as the fluidic module 9. In general the optical elements or unit are adjusted relative to the deformable cover 2. Such optical elements or units may be used in combination with light emitters 3, which are arranged in a reusable unit of a medical fluid delivery system and emit light beams perpendicular to the deformable cover 2. On the reflection side the reflected light beams may be deflected by the optical elements or unit such, that the reflected light beam perpendicularly enters the reusable unit or the optical window 8 thereof.

Using optical elements allows that the transition of the light beams between a disposable and a reusable unit of a medical fluid delivery system is in general perpendicular, which facilitates positioning of disposable and reusable unit relative to each other, in particular along an orientation perpendicular to the interface surfaces. Furthermore the optoelectronic parts of the sensor device, like the light emitter and the sensor unit, arranged in the reusable unit, can be positioned also in small and narrow constructions of a reusable unit.

In general it is possible to select only a part of the light emitted by the light emitter 3, which corresponds to the direction focusing on the inflexion point area 12 of the cover 2. Thus the lateral position of the disposable unit relative to the reusable unit along the surface of the deformable cover allows more flexibility and tolerances are larger. For example a light-shaping element may be used to confine the emitted light beam.

In figure 4 a first embodiment of a sensor device according to the disclosure is shown using an optical unit 13 to direct the incident and the reflected light beams to and from the inflection point area 12 of the deformable cover 2. The light path of the light beam according to a non-pressurized state is shown as a full line. The light beam 4' emitted by the light emitter 3 enters the optical unit 13 perpendicularly and is reflected at an optical element 14 within the optical unit 13 comprising an angled reflection surface, such that an incident light beam 4 exits the optical unit 13 at an angle $\alpha$ towards the deformable cover 2. A mirror optical element could e. g. be used. An exiting surface 15 is designed such, that it is orientated perpendicular to the incident light beam 4. Alternatively the surface could be designed as a refracting optical element and such contribute to the alignment of the incident light beam. The incident light beam 4 is reflected at the inflexion point area 12. The reflected light beams 6 and 6' are directed back to the optical unit 13, enter the optical unit 13 generally perpendicular and are reflected at a second optical element 14' within the optical element 13. The reflected light beam 6" according to a non-pressurized state is reflected on the optical element 14', exits the optical element 13 and perpendicularly meets the first photodiode 5a of the sensor unit 5. The light path according to a pressurized state is shown as pointed line. The deformation of the deformable cover 2 deflects the reflected light beam 6' as explained above. Therefore the reflected light beam 6' is reflected at the optical element 14' offset from the reflected light beam 6 and is guided towards the photodiode 5b as reflected light beam 6"'.

In figure 5 a second embodiment of a sensor device is shown, which is similar to one of figure 4. But the optical elements for guiding the light beams are separated from each other instead of being part of an optical unit13. A first optical element 14 is used to reflect the light beam 4' of the light emitter 3. A second optical element 14' is used to reflect the reflected light beam 6 in direction to photodiode 5a. And a third optical element 14" is used to reflect the shifted reflected light beam 6' in direction to photodiode 5b. The single optical elements 14, 14' and 14" can be oriented individually from each other.

In figure 6 a third embodiment of a sensor device is shown, which comprises an optical unit 13 with optical elements 14 and 14' in form of a convex free-forming surface. A bundle of light beams 4' emitted by the light emitter 3 is reflected at the optical element 14 and simultaneously the bundle of light beams 4' is focused on the inflexion point area 12 on the deformable cover 2 by the convex form of the free forming surface. The reflected light beams 6 and 6' are reflected at the convex free-forming surface of the optical element 14'. Due to the convex form the reflected light beam 6" (non-pressurized state) and also the reflected light beam 6"' (pressurized state) run parallel to each other and perpendicular to the sensor unit 5. The angle $\gamma$ at the reflection point of the optical element 14' to focus the reflected light beams 6" and 6"' on the sensor unit 5 is for example defined as $\gamma = 45° - \frac{1}{2}(\alpha + \delta)$.

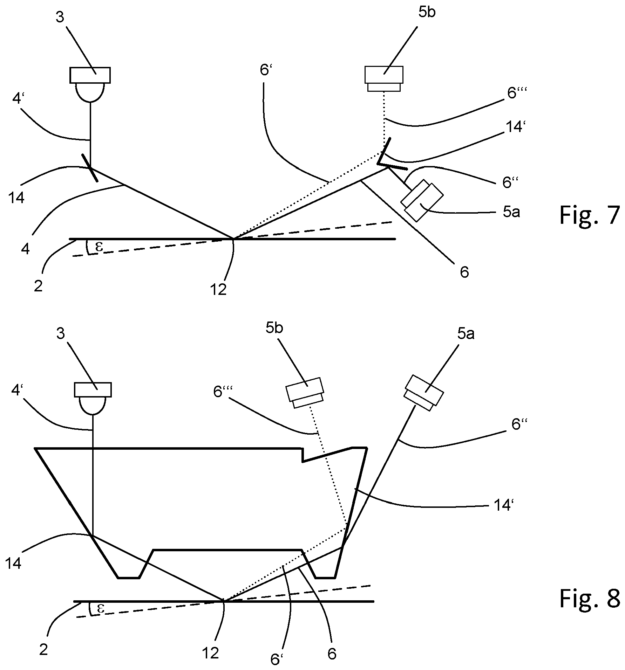

The sensor devices as shown in figures 7 and 8 are advantageously used for the detection of an occlusion of a medical fluid delivery system. The reflected light beam reflected from the deformable cover 2 is deflected at an optical element in largely different directions for a reflected light beam 6 reflected at a non-deformed cover 2 and for a reflected light beam 6' reflected at a deformed cover 2. That means the photo-elements 5a and 5b of the dual-element sensor 5 can be arranged individually and apart from each other.

In figure 7 the incident light beam is directed to the deformable cover 2 as shown in figure 5. The reflected light beams 6 and 6' are directed to an angled optical element 14' comprising two reflecting planes, which are orientated in an angle towards each other. In a non-pressurized state the reflected light beam 6 is directed to a first plane of the optical element 14', which reflects the reflected light beam 6" to the photodiode 5a. In a pressurized state the reflected light beam 6' is directed to a second plane of the optical element 14', which reflects the reflected light beam 6'" to the photodiode 5b. The reflected light beams 6" and 6'" are nearly directed in opposite directions. The angle between first and second plane may be adapted to a desired location of the photodiodes 5a and 5b or according to space requirements in the reusable unit.

In figure 8 the incident light beam is directed to an optical unit 13, which comprises an optical element 14' in form of an optical surface, which acts as a refraction surface for reflected light beams reflected in a non deformed state of the deformable cover 2 and acts as a reflection surface for reflected light beams reflected in a deformed state of the deformable cover 2. In particular the optical surface may be designed such that the reflected light beam is reflected at the optical element 14' as soon as the deformation of the deformable cover 2 corresponds to the occlusion threshold value indicating an occlusion condition of the fluidic module 9. Also the sensor unit 5 may comprise a third photodiode, which is energized by a light beam reflected on the deformable cover 2 in a state in between the non-pressurized state and the pressurized state and is refracted at the optical surface in direction of the third photodiode.

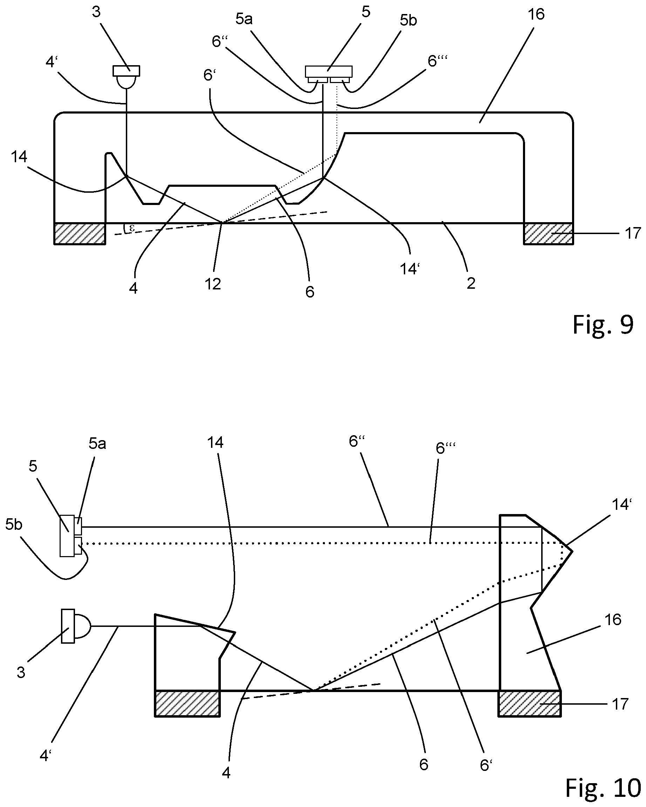

In figures 9 and 10 sensor devices for use in a medical fluid delivery system are shown, wherein optical elements 14 and 14' are part of a cap or covering 16 of the fluidic module 9, which is attached to a surrounding edge 17 of the fluidic module. As shown in figure 9 the covering 16 extends between the optoelectronic parts arranged in the reusable unit of the medical fluid delivery system and the deformable cover 2 and stretches between the edges 17. In direction towards the deformable cover 2 the covering 16 is designed according to an optical unit as shown in figures 4 or 6 respectively and provides optical elements 14 and 14' to direct the incident light beams 4 and 4' and the reflected light beams 6, 6', 6" and 6'".

In figure 10 an embodiment of the sensor device according to the present disclosure is shown, wherein the light emitter 3 emits a light beam 4' and the sensor unit 5 receives reflected light beams 6" and 6'" parallel to cover 2. That means the optoelectronic parts in the reusable unit can be located on a side of the disposable unit instead of opposite to it. The fluidic module comprises optic elements 14 and 14', wherein a first prism 14 directs the light beam 4' of the light emitter 3 as an incident light beam 4 on the inflexion point area 12 of the deformable cover 2 and a second prism 14' in form of a double prism directs the reflected light beams 6" and 6'" to the sensor unit 5. As explained before the prism 14' is designed such, that the light beam 6 reflected in a non-pressurized state of the deformable cover 2 is directed to the photodiode 5a and the light beam 6' reflected in a pressurized state of the deformable cover 2 is directed to the photodiode 5b.

The present disclosure has been described in respect of several embodiments of a sensor device. The present invention is not to be limited in scope by the specific embodiments described herein. Of course the features according to specific described embodiments may be combined to further embodiments by a person skilled in the art although not explained in detail herein. Indeed, various modifications of the present invention, in addition to those described herein, will be apparent to those skilled in the art from the foregoing description and accompanying drawings. Thus, such modifications are intended to fall within the scope of the appended claims.

**List of Reference Numerals**

| 1 | fluidic chamber |
|---|---|
| 2 | deformable cover |
| 3 | light emitter |
| 4, 4' | incident light beam |
| 5 | sensor unit |
| 5a | first photodiode |
| 5b | second photodiode |
| 6, 6', 6", 6'" | reflected light beam |
| 7 | optical parallelizing element |
| 8 | optical window |
| 9 | fluidic module |
| 10 | fluid inlet |
| 11 | fluid outlet |
| 12 | inflexion point area |
| 13 | optical unit |
| 14, 14', 14" | optical element |
| 15 | exiting surface |
| 16 | covering |
| 17 | fluidic chamber edge |

**Claims**

1.  A sensor device for use in a medical fluid delivery system comprising a fluidic chamber (1) with a deformable cover (2) closing at least one area of the chamber and an optical detection system comprising at least one light emitter (3) for emitting one or more incident light beams (4) and a sensor unit (5) for monitoring one or more reflected light beams (6), **wherein**
the one or more incident light beams (6) emitted by the light emitter (3) are directed on the cover (2) such that the one or more incident light beams (4) are reflected at an inflexion point area (12) that is formed within the deformable cover (2) upon deformation of the deformable cover (2) in a pressurized state of the fluidic chamber (1) **characterized in that**:
the one or more incident light beams (4) are reflected at an inflection point area (12) located closest to the light emitter (3).

2. The sensor device according to claim 1, **characterized in that** the incident one or more light beams (4) are directed on the cover (2) at an angle α.

3. The sensor device according to any of the preceding claims, **characterized in that** the light emitter (3) of the sensor device is located on a side of the cover (2) which is curved in a convex manner in an increased pressure state of the fluidic chamber (1).

4. The sensor device according to any of the preceding claims, **characterized in that** one side of the cover (2) is in fluid contact and the light emitter (3) is located on the other side.

5. The sensor device according to any of the preceding claims, **characterized in that** the incident light beam (4) is essentially a parallel light beam.

6. The sensor device according to any of the preceding claims, **characterized in that** the light emitter (3) is a laser diode or a light emitting diode comprising an optical element for parallelizing emitted light beams.

7. The sensor device according to any of the preceding claims, **characterized in that** the sensor unit (5) is provided by a dual-element sensor with two photoelements (5a, 5b).

8. The sensor device according to claim 7, **characterized in that** an optical element (14') may be provided in an optical path from the inflexion point area towards one or both of first and/or second photo-elements (5a, 5b).

9. The sensor device according to any of the preceding claims, **characterized in that** a diameter of an over all incident light beam ($D_{lb}$) is selected such that an edge of the beam does not extend beyond a center point of the cover (2).

10. The sensor device according to any of the preceding claims, **characterized in that** the cover (2) covers an at least partially rounded opening of the fluidic chamber (1).

11. A medical fluid delivery system, **characterized by** a sensor device according to any of claims 1 to 10.

12. The medical fluid delivery system according to claim 11, **characterized in that** the optical detection system is part of a reusable unit of the delivery system and the fluidic chamber (1) is part of a disposable unit of the delivery system, wherein the disposable unit comprises an optical element (14) for directing the incident light beam (4) in an angle α on the inflexion point area (12) of the cover (2).

13. The medical fluid delivery system according to claim 12, **characterized in that** the disposable unit comprises an optical element (14') for directing the reflected light beam (6) from the inflexion point area (12) to the sensor unit (5).

14. A disposable unit for use with a medical fluid delivery system according to claim 12 or claim 13.

15. A method for monitoring a pressure change in a fluidic chamber (1) of a medical fluid delivery system using a sensor device according to any of claims 1 to 10, **characterized by**:

    - directing one or more incident light beams (4) on the inflexion point area (12) of the deformable cover (2) located closest to the light emitter (3),
    - detecting one or more reflected light beams (6) reflected from the deformable cover (2) in a nonpressurized state and a pressurized state, and
    - comparing detection data (S1, S2) of the nonpressurized state and the pressurized state to extract the pressure change value.

**Patentansprüche**

1. Sensorvorrichtung zur Verwendung in einem medizinischen Flüssigkeitsabgabesystem, die eine Flüssigkeitskammer (1) mit einer verformbaren Abdeckung (2), die mindestens einen Bereich der Kammer verschließt, und ein optisches Erfassungssystem umfasst, das mindestens einen Lichtsender (3) zum Aussenden von einem oder mehreren einfallenden Lichtstrahlen (4) und eine Sensoreinheit (5) zum Überwachen von einem oder mehreren reflektierten Lichtstrahl(en) (6) umfasst, wobei der eine oder die mehreren einfallenden Lichtstrahlen (6), der bzw. die von dem Lichtsender (3) ausgesendet wird bzw. werden, derart auf die Abdeckung (2) gerichtet wird bzw. werden, dass der eine oder die mehreren einfallenden Lichtstrahlen (4) in einem Wendepunktbereich (12) reflektiert wird bzw. werden, der bei Verformung der verformbaren Abdeckung (2) in einem unter Druck gesetzten Zustand der Flüssigkeitskammer (1) innerhalb der verformbaren Abdeckung (2) gebildet ist, **dadurch gekennzeichnet, dass**:

der eine oder die mehreren einfallenden Lichtstrahlen (4) in einem Wendepunktbereich (12) reflektiert wird bzw. werden, der dem Lichtsender (3) am nächsten liegt.

2. Sensorvorrichtung nach Anspruch 1, **dadurch gekennzeichnet, dass** der einfallende eine Lichtstrahl oder die einfallenden mehreren Lichtstrahlen (4) unter einem Winkel α auf die Abdeckung (2) gerichtet wird bzw. werden.

3. Sensorvorrichtung nach einem der vorhergehenden Ansprüche, **dadurch gekennzeichnet, dass** sich der Lichtsender (3) der Sensorvorrichtung auf einer Seite der Abdeckung (2) befindet, die in einem Zustand der Flüssigkeitskammer (1) mit erhöhtem Druck konvex gekrümmt ist.

4. Sensorvorrichtung nach einem der vorhergehenden Ansprüche, **dadurch gekennzeichnet, dass** eine Seite der Abdeckung (2) in Flüssigkeitskontakt steht und sich der Lichtsender (3) auf der anderen Seite befindet.

5. Sensorvorrichtung nach einem der vorhergehenden Ansprüche, **dadurch gekennzeichnet, dass** der einfallende Lichtstrahl (4) im Wesentlichen ein paralleler Lichtstrahl ist.

6. Sensorvorrichtung nach einem der vorhergehenden Ansprüche, **dadurch gekennzeichnet, dass** der Lichtsender (3) eine Laserdiode oder eine Leuchtdiode ist, die ein optisches Element zum Parallelisieren von ausgesendeten Lichtstrahlen umfasst.

7. Sensorvorrichtung nach einem der vorhergehenden Ansprüche, **dadurch gekennzeichnet, dass** die Sensoreinheit (5) von einem Zweifachelementsensor mit zwei Photoelementen (5a, 5b) bereitgestellt wird.

8. Sensorvorrichtung nach Anspruch 7, **dadurch gekennzeichnet, dass** ein optisches Element (14') in einem Lichtweg vom Wendepunktbereich zu einem oder beiden von dem ersten und/oder zweiten Photoelement (5a, 5b) vorgesehen sein kann.

9. Sensorvorrichtung nach einem der vorhergehenden Ansprüche, **dadurch gekennzeichnet, dass** ein Durchmesser eines gesamten einfallenden Lichtstrahls ($D_{lb}$) derart gewählt ist, dass ein Rand des Strahls nicht über einen Mittelpunkt der Abdeckung (2) hinaus verläuft.

10. Sensorvorrichtung nach einem der vorhergehenden Ansprüche, **dadurch gekennzeichnet, dass** die Abdeckung (2) eine zumindest teilweise abgerundete Öffnung der Flüssigkeitskammer (1) abdeckt.

11. Medizinisches Flüssigkeitsabgabesystem, das durch eine Sensorvorrichtung nach einem der Ansprüche 1 bis 10 gekennzeichnet ist.

12. Medizinisches Flüssigkeitsabgabesystem nach Anspruch 11, **dadurch gekennzeichnet, dass** das optische Erfassungssystem Bestandteil einer wiederverwendbaren Einheit des Abgabesystems ist und die Flüssigkeitskammer (1) Bestandteil einer wegwerfbaren Einheit des Abgabesystems ist, wobei die wegwerfbare Einheit ein optisches Element (14) zum Richten des einfallenden Lichtstrahls (4) unter einem Winkel α auf den Wendepunktbereich (12) der Abdeckung (2) umfasst.

13. Medizinisches Flüssigkeitsabgabesystem nach Anspruch 12, **dadurch gekennzeichnet, dass** die wegwerfbare Einheit ein optisches Element (14') zum Richten des reflektierten Lichtstrahls (6) vom Wendepunktbereich (12) zur Sensoreinheit (5) umfasst.

14. Wegwerfbare Einheit zur Verwendung mit einem medizinischen Flüssigkeitsabgabesystem nach Anspruch 12 oder Anspruch 13.

15. Verfahren zum Überwachen einer Druckänderung in einer Flüssigkeitskammer (1) eines medizinischen Flüssigkeitsabgabesystems unter Verwendung einer Sensorvorrichtung nach einem der Ansprüche 1 bis 10, **gekennzeichnet durch**:

    - Richten von einem oder mehreren einfallenden Lichtstrahlen (4) auf den Wendepunktbereich (12) der verformbaren Abdeckung (2), der dem Lichtsender (3) am nächsten liegt,
    - Erfassen von einem oder mehreren reflektierten Lichtstrahlen (6), der bzw. die von der verformbaren Abdeckung (2) in einem nicht unter Druck gesetzten Zustand und einem unter Druck gesetzten Zustand reflektiert wird bzw. werden, und
    - Vergleichen von Erfassungsdaten (S1, S2) zum nicht unter Druck gesetzten Zustand und zum unter Druck gesetzten Zustand zwecks Gewinnung des Druckänderungswerts.

**Revendications**

1. Dispositif de détection pour son utilisation dans un système de délivrance de fluide médical comprenant une chambre à fluide (1) dotée d'un capot déformable (2) fermant au moins une zone de la chambre et un système de détection optique comprenant au moins un émetteur lumineux (3) pour émettre un ou plusieurs faisceaux lumineux incidents (4) et une unité de détection (5) pour surveiller un ou plusieurs faisceaux lumineux réfléchis (6), dans lequel les un ou plusieurs faisceaux lumineux incidents (6) émis par les émetteurs lumineux (3) sont dirigés sur le capot (2) de manière à ce que les un ou plusieurs faisceaux lumineux incidents (4) soient réfléchis au niveau d'une zone de point d'inflexion (12) qui est formée dans le capot déformable (2) en cas de déformation du capot déformable (2) dans un état pressurisé de la chambre à fluide (1), **caractérisé en ce que** :

les un ou plusieurs faisceaux lumineux incidents (4) sont réfléchis au niveau d'une zone de point d'inflexion (12) située le plus proche de l'émetteur lumineux (3).

2. Dispositif de détection selon la revendication 1, **caractérisé en ce que** les un ou plusieurs faisceaux lumineux incidents (4) sont dirigés sur le capot (2) à un angle α.

3. Dispositif de détection selon l'une quelconque des revendications précédentes, **caractérisé en ce que** l'émetteur lumineux (3) du dispositif de détection se trouve sur un côté du capot (2) qui est incurvé de manière convexe dans un état de pression accrue de la chambre à fluide (1).

4. Dispositif de détection selon l'une quelconque des revendications précédente, **caractérisé en ce qu'**un côté du capot (2) est en contact fluidique et que l'émetteur lumineux (3) se trouve sur l'autre côté.

5. Dispositif de détection selon l'une quelconque des revendications précédentes, **caractérisé en ce que** le faisceau lumineux incident (4) est essentiellement un faisceau lumineux parallèle.

6. Dispositif de détection selon l'une quelconque des revendications précédentes, **caractérisé en ce que** l'émetteur lumineux (3) est une diode laser ou une diode électroluminescente comprenant un élément optique pour paralléliser les faisceaux lumineux émis.

7. Dispositif de détection selon l'une quelconque des revendications précédentes, **caractérisé en ce que** l'unité de détection (5) est pourvue d'un capteur à double élément doté de deux photoéléments (5a, 5b).

8. Dispositif de détection selon la revendication 7, **caractérisé en ce qu'**un élément optique (14') peut être fourni dans une trajectoire optique de la zone de point d'inflexion en direction de l'un ou des deux des premier et/ou second photoéléments (5a, 5b).

9. Dispositif de détection selon l'une quelconque des revendications précédentes, **caractérisé en ce qu'**un diamètre d'un faisceau lumineux incident global ($D_{Ib}$) est sélectionné de manière à ce qu'un bord du faisceau ne s'étende pas au-delà d'un point central du capot (2) .

10. Dispositif de détection selon l'une quelconque des revendications précédentes, **caractérisé en ce que** le capot (2) couvre une ouverture au moins partiellement arrondie de la chambre à fluide (1).

11. Système de délivrance de fluide médical, **caractérisé par** un dispositif de détection selon l'une quelconque des revendications 1 à 10.

12. Système de délivrance de fluide médical selon la revendication 11, **caractérisé en ce que** le système de détection optique fait partie d'une unité réutilisable du système de délivrance et la chambre à fluide (1) fait partie d'une unité jetable du système de délivrance, dans lequel l'unité jetable comprend un élément optique (14) pour diriger le faisceau lumineux incident (4) selon un angle α sur la zone de point d'inflexion (12) du capot (2).

13. Système de délivrance de fluide médical selon la revendication 12, **caractérisé en ce que** l'unité jetable comprend un élément optique (14') pour diriger le faisceau lumineux réfléchi (6) de la zone de point d'inflexion (12) jusqu'à l'unité de détection (5).

14. Unité jetable pour son utilisation avec un système de délivrance de fluide médical selon la revendication 12 ou la revendication 13.

15. Procédé de surveillance d'un changement de pression dans une chambre à fluide (1) d'un système de délivrance de fluide médical en utilisant un dispositif de détection selon l'une quelconque des revendications 1 à 10, **caractérisé par** :

- la direction d'un ou plusieurs faisceaux lumineux incidents (4) sur la une zone de point d'inflexion (12) du capot déformable (2) située au plus près de l'émetteur lumineux (3),
- la détection d'un ou de plusieurs faisceaux lumineux réfléchis (6) réfléchis par le capot déformable (2) dans un état non pressurisé et un état pressurisé, et
- la comparaison de données de détection (S1, S2) de l'état non pressurisé et de l'état pressurisé pour extraire la valeur de changement de pression.

Fig. 1a

Fig. 1b

Fig. 2a

Fig. 2b

Fig. 2c

Fig. 3a

Fig. 3b

Fig. 4

Fig. 5

Fig. 6

Fig. 7

Fig. 8

Fig. 9

Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1970677 A1
- WO 2007093064 A1
- WO 2008144693 A1
- FR 2540987 A1
- EP 2399626 A1

**Non-patent literature cited in the description**

- **M. J. KOHL et al.** A microfluidic experimental platform with internal pressure measurements. *Sensors and Actuators A,* 2005, vol. 118, 212-221